# EUROPÄISCHE PATENTSCHRIFT

(11) **EP 4 609 901 B1**
(45) Veröffentlichungstag und Bekanntmachung des Hinweises auf die Patenterteilung: **12.08.2026**
(21) Anmeldenummer: 25160536.6
(22) Anmeldetag: 27.02.2025
(51) Int. Cl.: A61M 16/10, A61M 16/00, F01N 1/00, G10K 11/16

(54) **ATEMTHERAPIEGERÄT**
RESPIRATORY THERAPY APPARATUS
APPAREIL DE THÉRAPIE RESPIRATOIRE

(30) Priorität: 01.03.2024 DE 102024105940
(43) Veröffentlichungstag der Anmeldung: 03.09.2025
(73) Patentinhaber: Löwenstein Medical Technology S.A., 2557 Luxembourg (LU)
(72) Erfinder: Gerlach, Angela, 22549 Hamburg (DE); Veers, Kathleen, 24116 Kiel (DE); Oster, Alexander, 24214 Gettorf (DE)
(74) Vertreter: Löwenstein Medical IP

(56) Entgegenhaltungen:
- WO-A1-2006/121139
- WO-A1-2013/147283
- WO-A1-2014/145912
- WO-A1-2020/024914
- CN-U- 205 055 109
- GB-A- 2 450 735
- JP-A- 2001 120 662
- JP-A- 2008 212 206
- US-A1- 2015 136 126
- US-B2- 8 028 695

## Beschreibung

### Technisches Gebiet

Die Erfindung betrifft ein Atemtherapiegerät.

### Stand der Technik

Ein Atemtherapiegerät kann zur Behandlung schlafbezogener Atmungsstörungen, beispielsweise eines obstruktiven Schlafapnoesyndroms, oder bei Beeinträchtigungen der Atempumpe, beispielsweise durch eine chronisch obstruktive Lungenerkrankung (COPD), eingesetzt werden. Zu diesem Zweck kann das Atemtherapiegerät beispielsweise einen dauerhaften Überdruck im Atemweg des Patienten erzeugen, auch CPAP *(continuous positive airway pressure)* genannt. Ein solches Atemtherapiegerät umfasst in der Regel ein Gebläse, das je nach Drehzahl deutlich hörbare Strömungsgeräusche verursachen kann. Solche Geräusche können insbesondere beim Schlafen als unangenehm oder sogar als störend wahrgenommen werden.

US 2015/136126 A1 offenbart eine Kammeranordnung, die mit einem Sauerstoffkonzentrator verbunden werden kann und zur Schalldämpfung geeignet ist. Dazu umfasst die Kammeranordnung zwei Kammern, die über einen rohrförmigen Kanal miteinander in Reihe geschaltet sind, sodass ein Atemgas von der ersten Kammer in die zweite Kammer strömen kann. Ferner umfasst die erste Kammer eine erste Öffnung und eine zweite Öffnung. Der Kanal umfasst einen von der ersten Öffnung ins Innere der ersten Kammer ragenden inneren Kanalabschnitt und einen von der ersten Öffnung in eine Außenumgebung der ersten Kammer (d. h. ins Innere der zweiten Kammer) ragenden äußeren Kanalabschnitt. Das Atemgas strömt - wenn es mittels eines Gebläses von einem Gaseinlass zu einem Gasauslass gefördert wird - von der zweiten Öffnung durch die erste Kammer zur ersten Öffnung und passiert dabei den Kanal.

Die folgenden Dokumente offenbaren weitere Kammeranordnungen zur Schalldämpfung: GB 2 450 735 A, WO 2013/147283 A1, WO 2006/121139 A1, US 8 028 695 B2, JP 2008 212206 A, WO 2014/145912 A1, JP 2001120662 A, CN 205 055 109 U und WO 2020/024914 A1.

### Offenbarung der Erfindung

Eine Aufgabe der Erfindung kann darin gesehen werden, ein Atemtherapiegerät bereitzustellen, mit dem Geräusche im Betrieb wirksam gedämpft werden können, sodass sie überhaupt nicht mehr oder zumindest nicht mehr als unangenehm oder störend wahrgenommen werden. Diese Aufgabe wird durch den Gegenstand des unabhängigen Anspruchs gelöst.

Vorteilhafte Ausführungsformen der Erfindung sind in den abhängigen Ansprüchen dargelegt.

Die Offenbarung betrifft ein Atemtherapiegerät, das einen Gaseinlass, einen Gasauslass, ein Gebläse zum Fördern von Atemgas vom Gaseinlass zum Gasauslass und eine Kammer zur Schalldämpfung umfasst. Die Kammer umfasst eine erste Öffnung, eine zweite Öffnung und einen rohrförmigen Kanal, der einen von der ersten Öffnung ins Innere der Kammer ragenden inneren Kanalabschnitt und/oder einen von der ersten Öffnung in eine Außenumgebung der Kammer ragenden äußeren Kanalabschnitt umfasst. Das Atemtherapiegerät ist so ausgebildet, dass das Atemgas, wenn es vom Gaseinlass zum Gasauslass gefördert wird, zwischen der ersten Öffnung und der zweiten Öffnung durch die Kammer strömt und dabei den Kanal, d. h. den inneren Kanalabschnitt und/oder den äußeren Kanalabschnitt, passiert.

Ein solches Atemtherapiegerät hat den Vorteil, dass unangenehme oder störende Betriebsgeräusche aufgrund der speziellen Schalldämpfungskammer deutlich abgeschwächt werden, insbesondere ohne dass dazu spezielle Schaumstoffdämmungen ins Gerät integriert zu werden brauchen. Dies kann die Herstellung, Wartung und Reinigung des Geräts vereinfachen.

Unter "Atemtherapiegerät" kann allgemein ein Beatmungsgerät zur invasiven oder nicht invasiven Beatmung oder ein Gerät zur Sekretentfernung, beispielweise in Form einer Hustenmaschine, verstanden werden. Das Atemtherapiegerät kann insbesondere für eine High-Flow-Therapie geeignet sein. Unter "Atemtherapiegerät" kann auch eine Komponente (von mehreren Komponenten) eines Beatmungsgeräts oder eines ein Beatmungsgerät umfassenden Beatmungssystems verstanden werden.

Der Gaseinlass und der Gasauslass können innerhalb des Atemtherapiegeräts über einen Strömungsweg miteinander verbunden sein. Der Strömungsweg kann beispielsweise mindestens eine der folgenden Komponenten umfassen: den Kanal, die Kammer, das Gebläse, eine Gasleitung, einen Luftbefeuchter, einen Gassensor. Der Gaseinlass kann mit einer geeigneten Atemgasquelle und/oder einer Außenumgebung des Atemtherapiegeräts verbindbar sein. Der Gasauslass kann mit einem Schlauch zum Versorgen eines Patienten mit dem Atemgas verbindbar sein. Der Schlauch kann beispielsweise an mindestens eine der folgenden Patientenschnittstellen angeschlossen sein: einen Tubus, eine Nasenmaske, eine Nasenkanüle, eine Gesichtsmaske. Der Gaseinlass und/oder der Gasauslass kann bzw. können beispielsweise in einem (Außen-)Gehäuse des Atemtherapiegeräts ausgebildet sein.

Unter "Gebläse" kann allgemein eine Strömungsmaschine zum Beaufschlagen des Atemgases mit einem Druck verstanden werden. Das Gebläse kann beispielsweise ein Druckverhältnis von 1,0 bis 1,3, von 1,3 bis 3,0 oder von mehr als 3,0 (jeweils von Druckseite zu Saugseite) erzeugen.

Die Kammer kann allgemein als ein Feder-Masse-System aufgefasst werden, das abhängig von seiner Abstimmung, insbesondere abhängig von einer Länge und/oder einem Strömungsquerschnitt des inneren Kanalabschnitts und/oder des äußeren Kanalabschnitts, Schall in relevanten Frequenzbereichen dämpfen kann. Das Innere der Kammer kann ein von mehreren Wänden - beispielsweise in einer Längenrichtung und/oder einer Höhenrichtung und/oder einer Breitenrichtung - begrenzter Hohlraum sein. Das Gebläse kann über seinen (saugseitigen) Eingang und/oder seinen (druckseitigen) Ausgang mit der ersten Öffnung und/oder der zweiten Öffnung unmittelbar und/oder mittelbar, beispielsweise mittels einer oder mehrerer Gasleitungen, fluidisch gekoppelt sein. Beispielsweise kann eine erste Gasleitung einerseits die erste Öffnung mit dem Eingang des Gebläses und andererseits die zweite Öffnung mit dem Gaseinlass verbinden, wobei eine zweite Gasleitung den Ausgang des Gebläses mit dem Gasauslass verbinden kann. Alternativ kann eine erste Gasleitung einerseits den Gaseinlass mit dem Eingang des Gebläses und andererseits die zweite Öffnung mit dem Ausgang des Gebläses verbinden, wobei eine zweite Gasleitung die erste Öffnung mit dem Gasauslass verbinden kann. Beide Ausführungsformen haben den Effekt, dass das Atemgas bei aktiviertem Gebläse von der zweiten Öffnung zur ersten Öffnung durch die Kammer strömt. Es ist aber auch eine Ausführungsform möglich, bei der das Atemgas bei aktiviertem Gebläse von der ersten Öffnung zur zweiten Öffnung strömt. Im Hinblick auf die schalldämpfende Wirkung hat es sich als besonders vorteilhaft erwiesen, wenn die Kammer auf der Saugseite des Gebläses, d. h. in Strömungsrichtung des Atemgases betrachtet vor dem Eingang des Gebläses, angeordnet ist.

Unter "rohrförmiger Kanal" kann vor- und nachstehend zweckmäßigerweise ein Kanal mit einer zumindest teilweise geschlossenen Querschnittsform verstanden werden, beispielsweise im Gegensatz zu einem rinnenförmigen Kanal mit einer offenen Querschnittsform. Der Querschnitt des rohrförmigen Kanals kann beispielsweise kreis-, ellipsen- oder quaderförmig sein. Möglich sind aber auch komplexere Querschnittsformen wie beispielsweise eine L- oder T-Form und/oder in Bezug auf die Längsrichtung des rohrförmigen Kanals variierende Querschnittsformen. Der rohrförmige Kanal kann beispielsweise als Rohr und/oder Schlauch ausgebildet sein.

Unter "Gasleitung" kann beispielsweise ein (starres) Rohr, ein Stutzen, ein Schlauch oder eine Kombination aus mindestens zwei dieser Beispiele verstanden werden.

Unter "Öffnung" wie in "erster Öffnung" oder "zweiter Öffnung" kann allgemein ein Durchgang durch eine Wand der Kammer verstanden werden. Der Durchgang kann insbesondere eine dem Inneren der Kammer zugewandte Innenfläche der Wand mit einer der Außenumgebung der Kammer zugewandten Außenfläche der Wand verbinden.

Die erste Öffnung und die zweite Öffnung können in Richtung einer Strömung des Atemgases durch die Kammer betrachtet zumindest teilweise einander gegenüberliegen, d. h. teilweise oder vollständig überlappen. Alternativ können die erste Öffnung und die zweite Öffnung zueinander versetzt angeordnet sein.

Es ist möglich, dass die erste Öffnung den Gaseinlass bildet und/oder die zweite Öffnung den Gasauslass bildet. Alternativ kann die erste Öffnung den Gasauslass bilden und/oder die zweite Öffnung den Gaseinlass bilden. Anders ausgedrückt kann bzw. können der Gaseinlass und/oder der Gasauslass durch eine oder mehrere Öffnungen oder Durchgänge in einer oder mehreren Wänden der Kammer gebildet sein. Dadurch können Druckverluste im Vergleich zu einer Ausführungsform, bei der die Kammer über eine oder mehrere Gasleitungen an den Gaseinlass und/oder den Gasauslass angeschlossen ist, deutlich verringert werden. Dies kann das Atemtherapiegerät im Betrieb effizienter und/oder leiser machen.

Unter "Kanalabschnitt" wie in "innerer Kanalabschnitt" oder "äußerer Kanalabschnitt" kann beispielsweise ein senkrecht oder schräg von einer Wand der Kammer abstehendes, ein- oder beidseitig verbundenes Rohrstück verstanden werden. Der innere Kanalabschnitt kann beispielsweise durch einen Luftspalt von der zweiten Öffnung und/oder einer Wand der Kammer, insbesondere einer der ersten Öffnung gegenüberliegenden Wand der Kammer, getrennt sein. Der Kanal oder mindestens einer der Kanalabschnitte kann in seiner Längsrichtung betrachtet gerade und/oder gekrümmt verlaufen. Ein gekrümmter Kanal(abschnitt) kann bewirken, dass der Atemgasstrom nicht senkrecht, sondern mehr oder weniger schräg auf die Kanalwand trifft, was Rauschen verringern kann.

Der innere Kanalabschnitt kann in seiner Länge mit dem äußeren Kanalabschnitt im Wesentlichen übereinstimmen oder maßgeblich, beispielsweise um mindestens 10 %, mindestens 30 % oder mindestens 50 %, vom äußeren Kanalabschnitt abweichen.

Im Folgenden werden verschiedene Ausführungsformen der Offenbarung beschrieben. Diese Ausführungsformen sind nicht als Beschränkung des Umfangs der Offenbarung zu verstehen.

Gemäß einer Ausführungsform kann der rohrförmige Kanal, genauer dessen innerer Hohlraum, zumindest abschnittsweise durch einen Wandabschnitt der Kammer und/oder eines sonstigen Gehäuses des Atemtherapiegeräts, beispielsweise dessen Außengehäuses, begrenzt sein.

Gemäß einer Ausführungsform kann der rohrförmige Kanal zumindest abschnittweise durch mindestens ein mit einem Gehäuse des Atemtherapiegeräts ohne Zuhilfenahme eines Werkzeugs kraft- und/oder formschlüssig verbindbares Steckteil begrenzt sein. In diesem Fall kann der rohrförmige Kanal zumindest abschnittsweise durch einen entsprechenden Spalt zwischen dem Gehäuse und dem Steckteil (oder den Steckteilen) gebildet sein. Dies kann die Reinigung des Atemtherapiegeräts erleichtern.

Gemäß einer Ausführungsform kann die zweite Öffnung der ersten Öffnung und/oder einem offenen Ende des inneren Kanalabschnitts in Richtung einer Strömung des Atemgases durch die Kammer betrachtet zumindest teilweise gegenüberliegen. Unter dem Begriff "offenes Ende" kann vor- und nachstehend insbesondere ein unverbundenes Ende verstanden werden.

Allgemeiner ausgedrückt kann mindestens eine der Öffnungen des rohrförmigen Kanals relativ zu mindestens einer der Öffnungen der Kammer, vorzugsweise einer Eintrittsöffnung der Kammer, mit einem gewissen Versatz angeordnet sein, sodass sich die jeweiligen Öffnungen teilweise (oder vollständig) überlappen. Dies hat den Effekt, dass unter Umständen weniger Schallwellen direkt durch die Kammer dringen.

Gemäß einer Ausführungsform kann ein (beispielsweise gemitteltes) Verhältnis R *= P*²/*A* zwischen 14 und 30, zwischen 16 und 30 oder zwischen 20 und 30 liegen, wobei P für einen (beispielsweise gemittelten) Umfang des rohrförmigen Kanals und *A* für eine (beispielsweise gemittelte) Querschnittsfläche des rohrförmigen Kanals steht. Mit solchen R-Werten - genauer aufgrund der damit einhergehenden signifikanten Erhöhung der Reibungsfläche für einen gegebenen Querschnitt im Vergleich zu anderen R-Werten - konnte in Versuchen eine besonders wirksame Schalldämpfung erzielt werden. In bestimmten Fällen ist auch ein R-Wert unter 14 (beispielsweise 12) oder über 30 (beispielsweise 40) denkbar.

Als besonders günstig hat sich auch eine Ausführungsform bewiesen, bei der der (beispielsweise gemittelte) Strömungsquerschnitt der Kammer zumindest in einem Abschnitt zwischen einer der Öffnungen der Kammer und einer der Öffnungen des rohrförmigen Kanals doppelt so breit oder mehr als doppelt so breit wie der (beispielsweise gemittelte) Strömungsquerschnitt des rohrförmigen Kanals ist.

Gemäß einer Ausführungsform kann das Atemtherapiegerät so ausgebildet sein, dass das Atemgas, wenn es vom Gaseinlass zum Gasauslass gefördert wird, von der zweiten Öffnung zur ersten Öffnung durch die Kammer strömt und dabei den Kanal passiert.

Gemäß einer Ausführungsform kann die zweite Öffnung einen anderen Strömungsquerschnitt, insbesondere einen deutlich größeren Strömungsquerschnitt, als die erste Öffnung und/oder als ein offenes Ende des inneren Kanalabschnitts haben. Die zweite Öffnung kann insbesondere dann einen deutlich größeren Strömungsquerschnitt als die erste Öffnung und/oder als das offene Ende des inneren Kanalabschnitts haben, falls das Atemgas - wenn es vom Gaseinlass zum Gasauslass gefördert wird - von der zweiten Öffnung zur ersten Öffnung durch die Kammer strömt. Umgekehrt kann die zweite Öffnung insbesondere dann einen deutlich kleineren Strömungsquerschnitt als die erste Öffnung und/oder als das offene Ende des inneren Kanalabschnitts haben, falls das Atemgas - wenn es vom Gaseinlass zum Gasauslass gefördert wird - von der ersten Öffnung zur zweiten Öffnung durch die Kammer strömt. Alternativ kann der Strömungsquerschnitt der zweiten Öffnung in seiner Größe und/oder Form mit dem Strömungsquerschnitt der ersten Öffnung und/oder des offenen Endes des inneren Kanalabschnitts im Wesentlichen übereinstimmen.

Gemäß einer Ausführungsform kann die zweite Öffnung bündig mit einer dem Inneren der Kammer zugewandten Innenfläche einer Wand der Kammer abschließen oder in nicht nennenswertem Ausmaß von der Innenfläche abstehen. Alternativ oder zusätzlich kann die zweite Öffnung bündig mit einer der Außenumgebung der Kammer zugewandten Außenfläche einer Wand der Kammer abschließen oder in nicht nennenswertem Ausmaß von der Außenfläche abstehen.

Ferner ist es möglich, dass die erste Öffnung an einer Seite bündig mit einer Innenfläche oder einer Außenfläche einer Wand der Kammer abschließt oder in nicht nennenswertem Ausmaß von der Innen- bzw. Außenfläche absteht.

Ein solcher ein- oder zweiseitiger bündiger Abschluss der ersten bzw. zweiten Öffnung hat den Vorteil, dass Druckverluste aufgrund der gleichgerichteten Strömung verringert werden können.

Gemäß einer Ausführungsform kann ein Strömungsquerschnitt des inneren Kanalabschnitts in seiner Größe und/oder Form mit einem Strömungsquerschnitt des äußeren Kanalabschnitts im Wesentlichen übereinstimmen.

Gemäß einer Ausführungsform können der innere Kanalabschnitt und der äußere Kanalabschnitt eine gemeinsame Längsachse haben. Die gemeinsame Längsachse des inneren Kanalabschnitts und des äußeren Kanalabschnitts kann gerade und/oder gekrümmt verlaufen. Alternativ können der innere Kanalabschnitt und der äußere Kanalabschnitt zueinander parallele Längsachse haben.

Gemäß einer Ausführungsform kann die Kammer in einer Längenrichtung einerseits durch eine erste Wand und andererseits durch eine zweite Wand begrenzt sein. In diesem Fall kann die erste Wand die erste Öffnung aufweisen und/oder die zweite Wand die zweite Öffnung aufweisen. Anders ausgedrückt können die erste Öffnung und die zweite Öffnung an einander gegenüberliegenden Seiten der Kammer angeordnet sein. Unter "Längenrichtung" kann allgemein eine erste, beispielsweise horizontale oder vertikale Raumrichtung in einem dreidimensionalen Koordinatensystem verstanden werden. Unter "Wand" wie in "erster Wand" oder "zweiter Wand" kann beispielsweise zumindest ein Abschnitt eines Bodens, einer Decke, einer Seitenwand oder eines Kammerdeckels der Kammer oder eine Kombination von mindestens zwei dieser Abschnitte verstanden werden. Beispielsweise kann die erste Wand einstückig mit dem inneren Kanalabschnitt und/oder dem äußeren Kanalabschnitt ausgebildet sein, z. B. in einem Spritzgießverfahren.

Gemäß einer Ausführungsform kann der innere Kanalabschnitt höchstens bis zur Hälfte einer (Gesamt-)Länge der Kammer in der Längenrichtung zwischen der ersten Wand und der zweiten Wand ins Innere der Kammer ragen. Die Länge der Kammer kann beispielsweise 30 mm bis 50 mm, bevorzugt 35 mm bis 45 mm, betragen. Alternativ oder zusätzlich kann eine (Gesamt-)Höhe der Kammer in einer zur Längenrichtung orthogonalen Höhenrichtung 20 mm bis 40 mm, bevorzugt 25 mm bis 35 mm, und/oder eine (Gesamt-)Breite der Kammer in einer zur Längenrichtung und zur Höhenrichtung orthogonalen Breitenrichtung 30 mm bis 50 mm, bevorzugt 35 mm bis 45 mm, betragen.

Gemäß einer Ausführungsform kann ein erstes Ende des inneren Kanalabschnitts mit der ersten Wand verbunden sein und ein zweites, unverbundenes Ende des inneren Kanalabschnitts ins Innere der Kammer ragen. Das zweite, unverbundene Ende des inneren Kanalabschnitts kann zusätzlich offen sein.

In entsprechender Weise kann ein erstes Ende des äußeren Kanalabschnitts mit der ersten Wand verbunden sein und ein zweites, verbundenes oder unverbundenes Ende des äußeren Kanalabschnitts in die Außenumgebung der Kammer ragen. Beispielsweise kann das zweite Ende des äußeren Kanalabschnitts unmittelbar oder mittelbar, z. B. mittels eines Stutzens und/oder eines Rohrs und/oder eines Schlauchs, an mindestens eine der folgenden Komponenten des Atemtherapiegeräts angeschlossen sein: den Gaseinlass, den Gasauslass, den Eingang des Gebläses, den Ausgang des Gebläses, eine zusätzliche Kammer zur Schalldämpfung, insbesondere eine zusätzliche Kammer, wie sie nachstehend näher beschrieben wird.

Gemäß einer Ausführungsform kann die Kammer ferner einen abnehmbaren und/oder beweglich, beispielsweise dreh- und/oder verschiebbar, gelagerten Kammerdeckel zum Verschließen der Kammer umfassen. Dies erleichtert den Zugang zum Inneren der Kammer, beispielsweise zu Wartungs-, Reinigungs- oder Reparaturzwecken.

Gemäß einer Ausführungsform kann der Kammerdeckel, wenn er die Kammer verschließt, zumindest einen Abschnitt der ersten Wand und/oder der zweiten Wand bilden.

Gemäß einer Ausführungsform kann die Kammer ferner ein Filtermaterial zum Filtern von Partikeln und/oder Feuchtigkeit aus dem zwischen der ersten Öffnung und der zweiten Öffnung durch die Kammer strömenden Atemgas umfassen. Das Filtermaterial kann im betriebsfähigen Zustand des Atemtherapiegeräts zumindest teilweise in einem Strömungsweg des Atemgases zwischen der ersten Öffnung und der zweiten Öffnung durch die Kammer angeordnet sein, sodass das Atemgas durch das Filtermaterial strömen kann. Das Filtermaterial kann insbesondere ein poröses Material sein. Beispielsweise kann das Filtermaterial einen Schaumstoff, ein gesintertes Metall, ein Drahtgeflecht, Fasern, Ton oder eine Kombination von mindestens zwei dieser Beispiele umfassen. Weitere Beispiele für geeignete Filtermaterialien sind Polyestervliese, Mischkunstfasern in einem Propylenträger oder synthetische Polyesterblends. Alternativ oder zusätzlich kann das Filtermaterial spezielle chemische und/oder physikalische Eigenschaften haben, durch die das Filtermaterial zusätzlich stark schalldämpfend wirkt. Somit kann die schalldämpfende Wirkung der Kammer weiter verbessert werden. Das Filtermaterial kann beispielsweise auf einem Träger zum Stabilisieren des Filtermaterials aufgebracht sein. Der Träger selbst kann ebenfalls gasdurchlässig sein. Beispielsweise kann der Träger als Gitter- und/oder Netzstruktur ausgebildet sein. Alternativ oder zusätzlich kann die Kammer an ihrer Innenwand mindestens ein Auflageelement zum Auflegen des Filtermaterials und/oder des Trägers aufweisen, beispielsweise in Form eines Vorsprungs oder eines Einrastelements.

Gemäß einer Ausführungsform kann das Filtermaterial zumindest teilweise das Innere der Kammer und/oder zumindest teilweise den Kanal ausfüllen. Insbesondere kann das Filtermaterial das Innere der Kammer und/oder des Kanals größtenteils oder vollständig ausfüllen. Dies ermöglicht eine effiziente Filterung, ohne dass die Bauform der Kammer und/oder des Atemtherapiegeräts in nennenswerter Weise angepasst werden muss.

Gemäß einer Ausführungsform kann das Filtermaterial der ersten Öffnung und/oder der zweiten Öffnung gegenüberliegend angeordnet sein. Dabei kann das Filtermaterial die jeweilige Öffnung berühren, beispielsweise luftdurchlässig abdecken, oder durch einen Luftspalt von der jeweiligen Öffnung getrennt sein.

Gemäß einer Ausführungsform kann das Filtermaterial als Teil eines in die Kammer und/oder den Kanal einschiebbaren Einschubelements ausgebildet sein. Dies ermöglicht ein einfaches Wechseln des Filtermaterials. Beispielsweise kann das Einschubelement als Teil des Kammerdeckels oder umgekehrt ausgebildet sein.

Gemäß einer Ausführungsform kann das Filtermaterial am Kammerdeckel befestigt sein und zusammen mit dem Kammerdeckel abnehmbar und/oder beweglich gelagert sein. Das Filtermaterial kann so am Kammerdeckel befestigt sein, dass es sich im Strömungsweg des Atemgases befindet, wenn der Kammerdeckel die Kammer verschließt. Dies erleichtert den Zugang zum Inneren der Kammer, beispielsweise zu Wartungs-, Reinigungs- oder Reparaturzwecken. Zudem ermöglicht dies ein einfaches Wechseln des Filtermaterials.

Gemäß einer Ausführungsform kann die Kammer ferner einen rohrförmigen weiteren Kanal umfassen. Der weitere Kanal kann einen von der zweiten Öffnung ins Innere der Kammer ragenden weiteren inneren Kanalabschnitt und/oder einen von der zweiten Öffnung in die Außenumgebung der Kammer ragenden weiteren äußeren Kanalabschnitt umfassen. In diesem Fall kann das Atemtherapiegerät so ausgebildet sein, dass das Atemgas ferner den weiteren Kanal passiert, wenn es vom Gaseinlass zum Gasauslass gefördert wird. Der weitere innere Kanalabschnitt kann in seiner Länge mit dem weiteren äußeren Kanalabschnitt im Wesentlichen übereinstimmen oder maßgeblich, beispielsweise um mindestens 10 %, mindestens 30 % oder mindestens 50 %, vom weiteren äußeren Kanalabschnitt abweichen.

Der Kanal kann in seiner Länge mit dem weiteren Kanal im Wesentlichen übereinstimmen oder maßgeblich, beispielsweise um mindestens 10 %, mindestens 30 % oder mindestens 50 %, vom weiteren Kanal abweichen.

Gemäß einer Ausführungsform kann ein erstes Ende des weiteren inneren Kanalabschnitts mit der zweiten Wand verbunden sein und ein zweites, unverbundenes Ende des weiteren inneren Kanalabschnitts ins Innere der Kammer ragen. Das zweite, unverbundene Ende des weiteren inneren Kanalabschnitts kann zusätzlich offen sein. Beispielsweise kann die zweite Wand einstückig mit dem weiteren inneren Kanalabschnitt und/oder dem weiteren äußeren Kanalabschnitt ausgebildet sein, z. B. in einem Spritzgießverfahren.

In entsprechender Weise kann ein erstes Ende des weiteren äußeren Kanalabschnitts mit der zweiten Wand verbunden sein und ein zweites, verbundenes oder unverbundenes Ende des weiteren äußeren Kanalabschnitts in die Außenumgebung der Kammer ragen. Beispielsweise kann das zweite Ende des weiteren äußeren Kanalabschnitts unmittelbar oder mittelbar, z. B. mittels eines Stutzens und/oder eines Rohrs und/oder eines Schlauchs, an mindestens eine der folgenden Komponenten des Atemtherapiegeräts angeschlossen sein: den Gaseinlass, den Gasauslass, den Eingang des Gebläses, den Ausgang des Gebläses, eine zusätzliche Kammer zur Schalldämpfung, insbesondere eine zusätzliche Kammer, wie sie nachstehend näher beschrieben wird.

Gemäß einer Ausführungsform kann ein offenes Ende des weiteren inneren Kanalabschnitts der ersten Öffnung und/oder einem offenen Ende des inneren Kanalabschnitts in Richtung einer Strömung des Atemgases durch die Kammer betrachtet zumindest teilweise gegenüberliegen.

Gemäß einer Ausführungsform kann ein offenes Ende des weiteren inneren Kanalabschnitts einen anderen Strömungsquerschnitt, insbesondere einen deutlich größeren Strömungsquerschnitt, als die erste Öffnung und/oder als ein offenes Ende des inneren Kanalabschnitts haben. Das offene Ende des weiteren inneren Kanalabschnitts kann insbesondere dann einen deutlich größeren Strömungsquerschnitt als die erste Öffnung und/oder als das offene Ende des inneren Kanalabschnitts haben, falls das Atemgas - wenn es vom Gaseinlass zum Gasauslass gefördert wird - von der zweiten Öffnung zur ersten Öffnung durch die Kammer strömt. Umgekehrt kann das offene Ende des weiteren inneren Kanalabschnitts insbesondere dann einen deutlich kleineren Strömungsquerschnitt als die erste Öffnung und/oder als das offene Ende des inneren Kanalabschnitts haben, falls das Atemgas - wenn es vom Gaseinlass zum Gasauslass gefördert wird - von der ersten Öffnung zur zweiten Öffnung durch die Kammer strömt. Alternativ kann der Strömungsquerschnitt des offenen Endes des weiteren inneren Kanalabschnitts in seiner Größe und/oder Form mit dem Strömungsquerschnitt der ersten Öffnung und/oder des offenen Endes des inneren Kanalabschnitts im Wesentlichen übereinstimmen.

Gemäß einer Ausführungsform kann eine Länge des weiteren inneren Kanalabschnitts höchstens ein Drittel einer Länge des inneren Kanalabschnitts betragen. Unter "Länge" kann eine Ausdehnung des jeweiligen Kanalabschnitts in dessen Längsrichtung, d. h. in Richtung dessen Längsachse, verstanden werden.

Gemäß einer Ausführungsform können der weitere innere Kanalabschnitt und der weitere äußere Kanalabschnitt eine gemeinsame Längsachse haben. Die gemeinsame Längsachse des weiteren inneren Kanalabschnitts und des weiteren äußeren Kanalabschnitts kann gerade und/oder gekrümmt verlaufen.

Gemäß einer Ausführungsform kann ein Strömungsquerschnitt des weiteren inneren Kanalabschnitts in seiner Größe und/oder Form mit einem Strömungsquerschnitt des weiteren äußeren Kanalabschnitts im Wesentlichen übereinstimmen. Alternativ kann der Strömungsquerschnitt des weiteren inneren Kanalabschnitts in seiner Größe und/oder Form maßgeblich vom Strömungsquerschnitt des weiteren äußeren Kanalabschnitts abweichen.

Gemäß einer Ausführungsform kann der Kanal einen anderen Strömungsquerschnitt, insbesondere einen deutlich kleineren Strömungsquerschnitt, als der weitere Kanal haben. Der Kanal kann insbesondere dann einen deutlich kleineren Strömungsquerschnitt als der weitere Kanal haben, falls das Atemgas - wenn es vom Gaseinlass zum Gasauslass gefördert wird - von der zweiten Öffnung zur ersten Öffnung durch die Kammer strömt. Umgekehrt kann der Kanal insbesondere dann einen deutlich größeren Strömungsquerschnitt als der weitere Kanal haben, falls das Atemgas - wenn es vom Gaseinlass zum Gasauslass gefördert wird - von der ersten Öffnung zur zweiten Öffnung durch die Kammer strömt.

Gemäß einer Ausführungsform kann eine Länge des weiteren Kanals höchstens ein Drittel einer Länge des Kanals betragen. Unter "Länge" kann eine Ausdehnung des jeweiligen Kanals in dessen Längsrichtung, d. h. in Richtung dessen Längsachse, verstanden werden.

Gemäß einer Ausführungsform können der Kanal und der weitere Kanal eine gemeinsame Längsachse haben. Die gemeinsame Längsachse des Kanals und des weiteren Kanals kann gerade und/oder gekrümmt verlaufen.

Gemäß einer Ausführungsform kann ein Volumen der Kammer 18 cm³ bis 160 cm³, insbesondere 30 cm³ bis 70 cm³, betragen.

Gemäß einer Ausführungsform kann ein Volumen des Kanals und/oder des inneren Kanalabschnitts 5 cm³ bis 12 cm³ betragen. Der Kanal kann in seinem Volumen mit dem weiteren Kanal im Wesentlichen übereinstimmen oder maßgeblich, beispielsweise um mindestens 10 %, mindestens 30 % oder mindestens 50 %, vom weiteren Kanal abweichen. Der innere Kanalabschnitt kann in seinem Volumen mit dem weiteren inneren Kanalabschnitt im Wesentlichen übereinstimmen oder maßgeblich, beispielsweise um mindestens 10 %, mindestens 30 % oder mindestens 50 %, vom weiteren inneren Kanalabschnitt abweichen.

Gemäß einer Ausführungsform kann eine größte Querschnittsfläche der Kammer 8 cm² bis 16 cm², insbesondere 10 cm² bis 14 cm², betragen.

Gemäß einer Ausführungsform kann eine größte Querschnittsfläche des Kanals und/oder des inneren Kanalabschnitts 1,8 cm² bis 2,0 cm² betragen. Der Kanal kann in seiner größten Querschnittsfläche mit dem weiteren Kanal im Wesentlichen übereinstimmen oder maßgeblich, beispielsweise um mindestens 10 %, mindestens 30 % oder mindestens 50 %, vom weiteren Kanal abweichen. Der innere Kanalabschnitt kann in seiner größten Querschnittsfläche mit dem weiteren inneren Kanalabschnitt im Wesentlichen übereinstimmen oder maßgeblich, beispielsweise um mindestens 10 %, mindestens 30 % oder mindestens 50 %, vom weiteren inneren Kanalabschnitt abweichen.

Mit solchen Maßen konnten in Versuchen besonders gute Ergebnisse in Bezug auf die schalldämpfende Wirkung erreicht werden.

Gemäß einer Ausführungsform kann das Atemtherapiegerät ferner eine zusätzliche Kammer zur Schalldämpfung umfassen. Ähnlich wie die vor- und nachstehend beschriebene Kammer kann die zusätzliche Kammer eine zusätzliche erste Öffnung, eine zusätzliche zweite Öffnung und einen rohrförmigen zusätzlichen Kanal umfassen, wobei der zusätzliche Kanal einen von der zusätzlichen ersten Öffnung ins Innere der zusätzlichen Kammer ragenden zusätzlichen inneren Kanalabschnitt und/oder einen von der zusätzlichen ersten Öffnung in eine Außenumgebung der zusätzlichen Kammer ragenden zusätzlichen äußeren Kanalabschnitt umfassen kann. In diesem Fall kann das Atemtherapiegerät so ausgebildet sein, dass das Atemgas, wenn es vom Gaseinlass zum Gasauslass gefördert wird, ferner zwischen der zusätzlichen ersten Öffnung und der zusätzlichen zweiten Öffnung durch die zusätzliche Kammer strömt und dabei den zusätzlichen Kanal passiert.

Gemäß einer Ausführungsform kann die Kammer mit der zusätzlichen Kammer oder den zusätzlichen Kammern in Reihe geschaltet sein, sodass das Atemgas die miteinander in Reihe geschalteten Kammern nacheinander durchströmt, wenn es vom Gaseinlass zum Gasauslass gefördert wird.

Die Kammer kann beispielsweise über einen Durchgang in einer Trennwand und/oder über einen rohrförmigen Verbindungskanal mit der zusätzlichen Kammer oder den zusätzlichen Kammern fluidisch verbunden sein. Der Verbindungskanal kann beispielsweise zumindest einen Abschnitt des Kanals und/oder des zusätzlichen Kanals und/oder des weiteren Kanals umfassen.

Beispielsweise können die verschiedenen Kammern akustisch unterschiedlich abgestimmt sein, sodass jede der Kammern einen anderen Frequenzbereich des Schalls dämpft.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt ein Atemtherapiegerät gemäß einer Ausführungsform der Offenbarung.
Fig. 2 zeigt eine Kammer eines Atemtherapiegeräts gemäß einer Ausführungsform der Offenbarung mit einem Einschubelement.
Fig. 3 zeigt eine Kammer eines Atemtherapiegeräts gemäß einer Ausführungsform der Offenbarung mit zueinander versetzten Öffnungen.
Fig. 4 zeigt eine Kammer eines Atemtherapiegeräts gemäß einer Ausführungsform der Offenbarung mit einem mit Filtermaterial ausgefüllten Kanal.
Fig. 5 zeigt eine Kammer eines Atemtherapiegeräts gemäß einer Ausführungsform der Offenbarung mit zwei Kanälen, die jeweils ins Innere der Kammer ragen.
Fig. 6 zeigt eine Kammer eines Atemtherapiegeräts gemäß einer Ausführungsform der Offenbarung mit zwei Kanälen, von denen nur einer ins Innere der Kammer ragt.
Fig. 7 zeigt eine Kammer eines Atemtherapiegeräts gemäß einer Ausführungsform der Offenbarung mit zwei Kanälen, von denen nur einer in eine Außenumgebung der Kammer ragt.
Fig. 8 zeigt zwei miteinander in Reihe geschaltete Kammern eines Atemtherapiegeräts gemäß einer Ausführungsform der Offenbarung mit asymmetrischem Aufbau.
Fig. 9 zeigt zwei miteinander in Reihe geschaltete Kammern eines Atemtherapiegeräts gemäß einer Ausführungsform der Offenbarung mit symmetrischem Aufbau.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Offenbarung

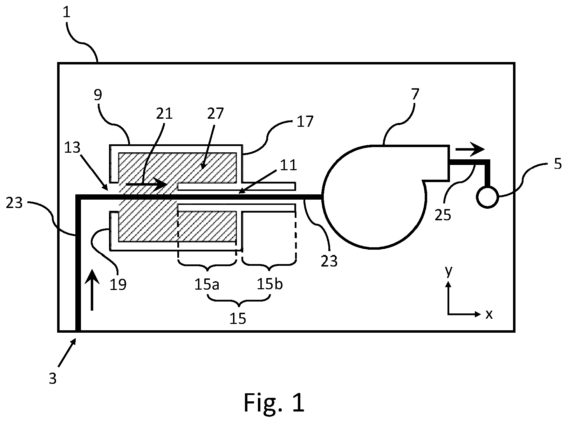

Fig. 1 zeigt ein Atemtherapiegerät 1, das einen Gaseinlass 3, einen Gasauslass 5, ein Gebläse 7 zum Fördern von Atemgas vom Gaseinlass 3 zum Gasauslass 5 und eine Kammer 9 zur Schalldämpfung umfasst.

Beispielsweise kann der Gaseinlass 3 mit einer geeigneten Atemgasquelle verbindbar sein. Alternativ oder zusätzlich kann der Gasauslass 5 über einen Schlauch mit einer entsprechenden Patientenschnittstelle, beispielsweise einer Nasenmaske, einer Gesichtsmaske, einer (High-Flow-)Nasenkanüle oder einem Tubus, verbindbar sein.

Die Kammer 9 umfasst eine erste Öffnung 11, eine zweite Öffnung 13 und einen rohrförmigen Kanal 15. Der Kanal 15 umfasst in diesem Beispiel einen von der ersten Öffnung 11 ins Innere der Kammer 9 ragenden inneren Kanalabschnitt 15a und einen von der ersten Öffnung 11 in eine Außenumgebung der Kammer 9 ragenden äußeren Kanalabschnitt 15b.

Das Atemtherapiegerät 1 ist so ausgebildet, dass das Atemgas, wenn es vom Gaseinlass 3 zum Gasauslass 5 gefördert wird, zwischen der ersten Öffnung 11 und der zweiten Öffnung 13, hier von der zweiten Öffnung 13 zur ersten Öffnung 11, durch die Kammer 9 strömt und dabei den Kanal 15 passiert.

Wie in Fig. 1 beispielhaft gezeigt, kann die Kammer 9 in einer Längenrichtung x einerseits durch eine erste Wand 17 mit der ersten Öffnung 11 und andererseits durch eine zweite Wand 19 mit der zweiten Öffnung 13 begrenzt sein. Dabei kann ein Abstand zwischen den beiden Wänden 17, 19 in der Längenrichtung x einer Länge der Kammer 9 entsprechen.

In diesem Beispiel ist ein erstes Ende des inneren Kanalabschnitts 15a mit der ersten Wand 17 verbunden, während ein zweites, unverbundenes Ende des inneren Kanalabschnitts 15a ins Innere der Kammer 9 ragt. Das zweite Ende des inneren Kanalabschnitts 15a kann offen sein und der zweiten Öffnung 13 in der zweiten Wand 19 in Richtung einer Strömung 21 des Atemgases von der zweiten Öffnung 13 zur ersten Öffnung 11 betrachtet teilweise oder vollständig gegenüberliegen.

In entsprechender Weise kann ein erstes Ende des äußeren Kanalabschnitts 15b mit der ersten Wand 17 verbunden sein, während ein zweites Ende des äußeren Kanalabschnitts 15b in die Außenumgebung der Kammer 9 ragen kann. Das zweite Ende des äußeren Kanalabschnitts 15b kann unmittelbar oder mittelbar mit einem (saugseitigen) Eingang des Gebläses 7 fluidisch verbunden sein. In diesem Beispiel ist das zweite Ende mittels einer ersten Gasleitung 23 mit dem Eingang verbunden. Ein (druckseitiger) Ausgang des Gebläses 7 kann beispielsweise mittels einer zweiten Gasleitung 25 mit dem Gasauslass 5 fluidisch verbunden sein.

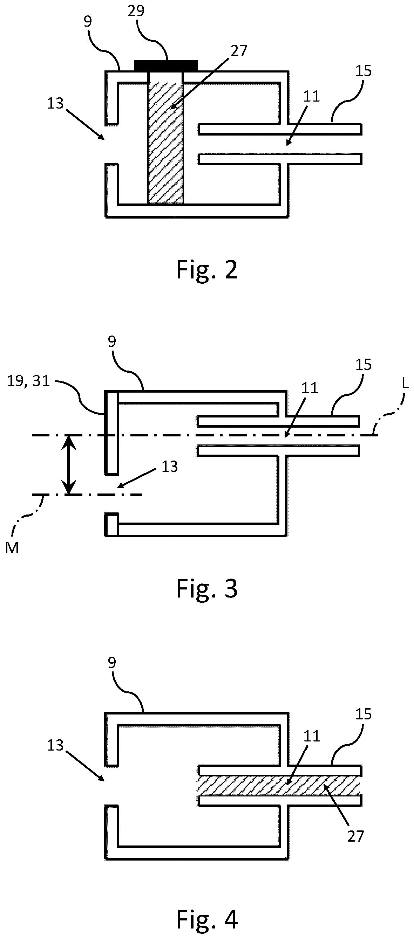

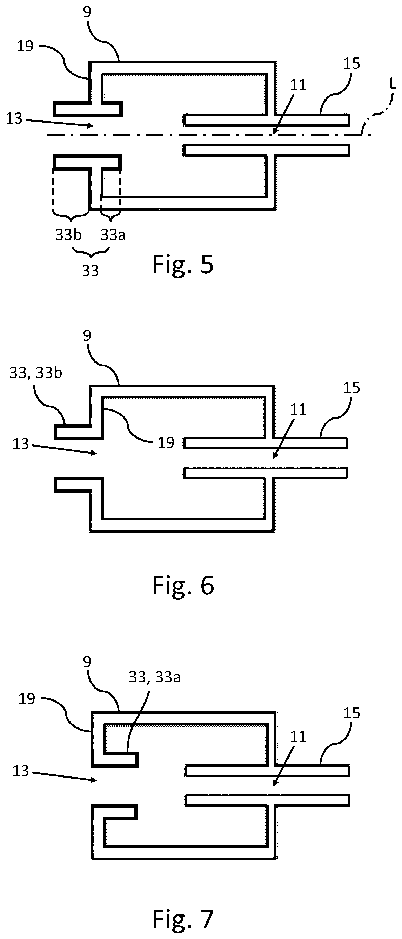

Wie in Fig. 1 zu sehen, kann die zweite Öffnung 13 ein einfacher Durchgang durch die zweite Wand 19 sein. Dabei kann eine Mittelachse der zweiten, beispielsweise runden Öffnung 13 auf einer Längsachse des Kanals 15 liegen (siehe auch Fig. 5) oder parallel dazu ausgerichtet sein (siehe auch Fig. 3).

Zusätzlich kann die Kammer 9 ein gasdurchlässiges Filtermaterial 27 zum Filtern von Partikeln und/oder Feuchtigkeit aus dem Atemgas umfassen. Das Filtermaterial 27 kann in einem Strömungsweg zwischen der ersten Öffnung 11 und der zweiten Öffnung 13 im Inneren der Kammer 9 und/oder im Kanal 15 angeordnet sein. Die Kammer 9 kann größtenteils oder sogar vollständig mit dem Filtermaterial 27 ausgefüllt sein. In Fig. 1. ist der Kanal 15 frei von jeglichem Filtermaterial 27.

Fig. 2 zeigt eine Ausführungsform der Kammer 9 mit einem optionalen Einschubelement 29, an dem das Filtermaterial 27 befestigt ist. In diesem Beispiel ist das Einschubelement 29 in einer zur Längenrichtung x orthogonalen Höhenrichtung y ins Innere der Kammer 9 einschiebbar. Dies ermöglicht einen einfachen Wechsel des Filtermaterials 27.

Fig. 3 zeigt eine Ausführungsform der Kammer 9, bei der die Längsachse L des Kanals 15 und die Mittelachse M der zweiten Öffnung 13 in der Höhenrichtung y zueinander versetzt sind (markiert mit einem Doppelpfeil). Die Kammer 9 umfasst hier zusätzlich einen abnehmbaren Kammerdeckel 31, der die zweite Wand 19 bildet, wenn er die Kammer 9 verschließt.

Fig. 4 zeigt eine Ausführungsform der Kammer 9, bei der das Filtermaterial 27 ausschließlich im Kanal 15 angeordnet ist. Das Filtermaterial 27 kann den Kanal 15 teilweise oder, wie hier, vollständig ausfüllen.

Fig. 5 zeigt eine Ausführungsform der Kammer 9 mit einem weiteren Kanal 33, der einen von der zweiten Wand 19 ins Innere der Kammer 9 ragenden weiteren inneren Kanalabschnitt 33a und/oder einen von der zweiten Wand 19 in die Außenumgebung der Kammer 9 ragenden weiteren äußeren Kanalabschnitt 33b umfassen kann. Der Kanal 15 und der weitere Kanal 33 können eine gemeinsame Längsachse L haben. Zudem kann der weitere Kanal 33 einen deutlich größeren Strömungsquerschnitt als der Kanal 15 haben. Abhängig von der Strömungsrichtung des Atemgases kann der weitere Kanal 33 auch einen deutlich kleineren Strömungsquerschnitt als der Kanal 15 haben. Alternativ oder zusätzlich können der Kanal 15 und der weitere Kanal 33 in ihrer Länge deutlich voneinander abweichen. Beispielsweise kann eine Länge des weiteren Kanals 33 nur höchstens ein Drittel einer Länge des Kanals 15 betragen. Ein derartiges Längenverhältnis kann dazu beitragen, Druckverluste zu verringern und/oder die schalldämpfende Wirkung der Kammer 9 weiter zu verbessern.

Fig. 6 zeigt eine Ausführungsform der Kammer 9, bei der der weitere Kanal 33 im Unterschied zur Ausführungsform von Fig. 5 nur den weiteren äußeren Kanalabschnitt 33b umfasst. In diesem Fall kann die zweite Öffnung 13 mit einer dem Inneren der Kammer 9 zugewandten Innenfläche der zweiten Wand 19 im Wesentlichen bündig abschließen.

Fig. 7 zeigt eine Ausführungsform der Kammer 9, bei der der weitere Kanal 33 im Unterschied zur Ausführungsform von Fig. 5 nur den weiteren inneren Kanalabschnitt 33a umfasst. In diesem Fall kann die zweite Öffnung 13 mit einer der Außenumgebung der Kammer 9 zugewandten Außenfläche der zweiten Wand 19 im Wesentlichen bündig abschließen.

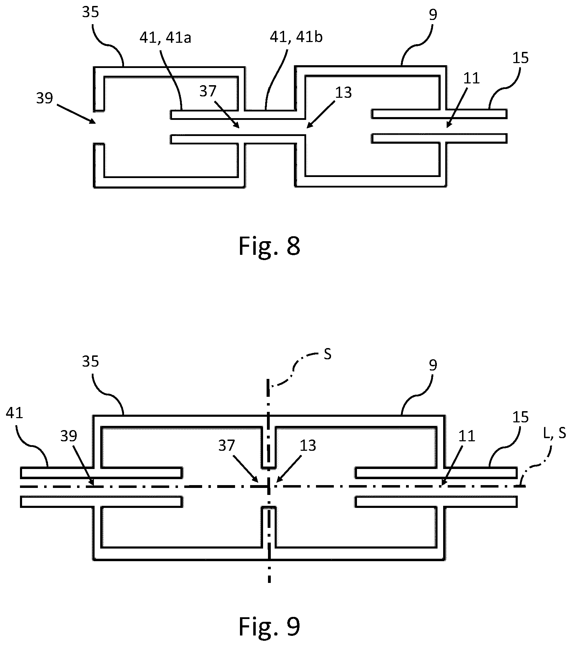

Fig. 8 zeigt eine Ausführungsform, bei der die Kammer 9 mit einer zusätzlichen Kammer 35 zur Schalldämpfung in Reihe geschaltet ist. Ähnlich wie die Kammer 9 kann die zusätzliche Kammer 35 eine zusätzliche erste Öffnung 37, eine zusätzliche zweite Öffnung 39 und einen rohrförmigen zusätzlichen Kanal 41 umfassen. Der zusätzliche Kanal 41 kann einen von der zusätzlichen ersten Öffnung 37 ins Innere der zusätzlichen Kammer 35 ragenden zusätzlichen inneren Kanalabschnitt 41a und/oder einen von der zusätzlichen ersten Öffnung 37 in eine Außenumgebung der zusätzlichen Kammer 35 ragenden zusätzlichen äußeren Kanalabschnitt 41b umfassen. Das Atemtherapiegerät kann so ausgebildet sein, dass das Atemgas, wenn es vom Gaseinlass zum Gasauslass gefördert wird, ferner zwischen der zusätzlichen ersten Öffnung 37 und der zusätzlichen zweiten Öffnung 39, beispielsweise von der zusätzlichen zweiten Öffnung 39 zur zusätzlichen ersten Öffnung 37, durch die zusätzliche Kammer 35 strömt und dabei den zusätzlichen Kanal 41 passiert.

In diesem Beispiel ist die zusätzliche erste Öffnung 37 über den zusätzlichen äußeren Kanalabschnitt 41b mit der zweiten Öffnung 13 der Kammer 9 fluidisch verbunden, sodass das Atemgas nacheinander durch die Kammern 9, 35 strömt, wenn es vom Gaseinlass zum Gasauslass gefördert wird.

Auf diese Weise kann die schalldämpfende Wirkung weiter verbessert werden. Beispielsweise können die verschiedenen Kammern 9, 35 akustisch unterschiedlich abgestimmt sein, sodass jede der Kammern 9, 35 einen anderen Frequenzbereich des Schalls dämpft.

Die Kammer 9 kann auch mit mehr als einer zusätzlichen Kammer 35, beispielsweise mit mindestens zwei oder mindestens vier zusätzlichen Kammern 35, in Reihe geschaltet sein.

Fig. 9 zeigt eine Ausführungsform, bei der der zusätzliche Kanal 41 im Unterschied zur Ausführungsform von Fig. 8 an der zusätzlichen zweiten Öffnung 39 angeordnet ist. In diesem Fall können die beiden Kammern 9, 35 über einen einfachen Durchgang in einer gemeinsamen Trennwand fluidisch miteinander verbunden sein. Der Durchgang kann sowohl die zusätzliche erste Öffnung 37 der zusätzlichen Kammer 35 als auch die zweite Öffnung 13 der Kammer 9 bilden. Beispielsweise können die beiden Kammern 9, 35 in Bezug auf eine gedachte Symmetrielinie S in vertikaler und/oder horizontaler Richtung symmetrisch ausgebildet sein.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Atemtherapiegerät
- 3: Gaseinlass
- 5: Gasauslass
- 7: Gebläse
- 9: Kammer
- 11: erste Öffnung
- 13: zweite Öffnung
- 15: rohrförmiger Kanal
- 15a: innerer Kanalabschnitt
- 15b: äußerer Kanalabschnitt
- 17: erste Wand
- 19: zweite Wand
- 21: Strömung
- 23: erste Gasleitung
- 25: zweite Gasleitung
- 27: Filtermaterial
- 29: Einschubelement
- 31: Kammerdeckel
- 33: rohrförmiger weiterer Kanal
- 33a: weiterer innerer Kanalabschnitt
- 33b: weiterer äußerer Kanalabschnitt
- 35: zusätzliche Kammer
- 37: zusätzliche erste Öffnung
- 39: zusätzliche zweite Öffnung
- 41: rohrförmiger zusätzlicher Kanal
- 41a: zusätzlicher innerer Kanalabschnitt
- 41b: zusätzlicher äußerer Kanalabschnitt
- *x*: Längenrichtung
- *y*: Höhenrichtung
- *L*: Längsachse
- *M*: Mittelachse
- *S*: Symmetrieachse

## Patentansprüche

1. Atemtherapiegerät (1), umfassend:
einen Gaseinlass (3);
einen Gasauslass (5);
ein Gebläse (7) zum Fördern von Atemgas vom Gaseinlass (3) zum Gasauslass (5);
eine Kammer (9) zur Schalldämpfung, wobei die Kammer (9) eine erste Öffnung (11), eine zweite Öffnung (13) und einen rohrförmigen Kanal (15) umfasst, wobei der Kanal (15) einen von der ersten Öffnung (11) ins Innere der Kammer (9) ragenden inneren Kanalabschnitt (15a) und einen von der ersten Öffnung (11) in eine Außenumgebung der Kammer (9) ragenden äußeren Kanalabschnitt (15b) umfasst, wobei die zweite Öffnung (13) einem offenen Ende des inneren Kanalabschnitts (15a) in Richtung einer Strömung (21) des Atemgases durch die Kammer (9) betrachtet zumindest teilweise gegenüberliegt, wobei die Kammer (9) in einer Längenrichtung (x) einerseits durch eine erste Wand (17) und andererseits durch eine zweite Wand (19) begrenzt ist, wobei die erste Wand (17) die erste Öffnung (11) aufweist und die zweite Wand (19) die zweite Öffnung (13) aufweist, wobei ein erstes Ende des inneren Kanalabschnitts (15a) mit der ersten Wand (17) verbunden ist und ein zweites, unverbundenes Ende des inneren Kanalabschnitts (15a) als das offene Ende des inneren Kanalabschnitts (15a) ins Innere der Kammer (9) ragt;
wobei das Atemtherapiegerät (1) so ausgebildet ist, dass das Atemgas, wenn es vom Gaseinlass (3) zum Gasauslass (5) gefördert wird, von der zweiten Öffnung (13) zur ersten Öffnung (11) durch die Kammer (9) strömt und dabei den Kanal (15) passiert;
wobei die zweite Öffnung (13) ein Durchgang durch die zweite Wand (19) ist, wobei der Durchgang eine dem Inneren der Kammer (9) zugewandte Innenfläche der zweiten Wand (19) mit einer der Außenumgebung der Kammer (9) zugewandten Außenfläche der zweiten Wand (19) verbindet, wobei die zweite Öffnung (13) einen größeren Strömungsquerschnitt als das offene Ende des inneren Kanalabschnitts (15a) hat;
**dadurch gekennzeichnet, dass** die zweite Öffnung (13) sowohl bündig mit der Innenfläche der zweiten Wand (19) als auch bündig mit der Außenfläche der zweiten Wand (19) abschließt.

2. Atemtherapiegerät (1) nach Anspruch 1,
wobei die zweite Öffnung (13) ferner der ersten Öffnung (11) in Richtung der Strömung (21) des Atemgases durch die Kammer (9) betrachtet zumindest teilweise gegenüberliegt; und/oder
wobei die zweite Öffnung (13) ferner einen anderen Strömungsquerschnitt, insbesondere einen größeren Strömungsquerschnitt, als die erste Öffnung (11) hat.

3. Atemtherapiegerät (1) nach einem der vorhergehenden Ansprüche,
wobei ein Strömungsquerschnitt des inneren Kanalabschnitts (15a) in seiner Größe und/oder Form mit einem Strömungsquerschnitt des äußeren Kanalabschnitts (15b) übereinstimmt; und/oder
wobei der innere Kanalabschnitt (15a) und der äußere Kanalabschnitt (15b) eine gemeinsame Längsachse (L) haben.

4. Atemtherapiegerät (1) nach einem der vorhergehenden Ansprüche,
wobei der innere Kanalabschnitt (15a) höchstens bis zur Hälfte einer Länge der Kammer (9) in der Längenrichtung (x) zwischen der ersten Wand (17) und der zweiten Wand (19) ins Innere der Kammer (9) ragt.

5. Atemtherapiegerät (1) nach einem der vorhergehenden Ansprüche,
wobei die Kammer (9) ferner einen abnehmbaren und/oder beweglich gelagerten Kammerdeckel (31) zum Verschließen der Kammer (9) umfasst.

6. Atemtherapiegerät (1) nach Anspruch 5,
wobei der Kammerdeckel (31), wenn er die Kammer (9) verschließt, zumindest einen Abschnitt der ersten Wand (17) und/oder der zweiten Wand (19) bildet.

7. Atemtherapiegerät (1) nach einem der vorhergehenden Ansprüche,
wobei die Kammer (9) ferner ein Filtermaterial (27) zum Filtern von Partikeln und/oder Feuchtigkeit aus dem zwischen der ersten Öffnung (11) und der zweiten Öffnung (13) durch die Kammer (9) strömenden Atemgas umfasst.

8. Atemtherapiegerät (1) nach Anspruch 7,
wobei das Filtermaterial (27) zumindest teilweise das Innere der Kammer (9) und/oder zumindest teilweise den Kanal (15) ausfüllt; und/oder
wobei das Filtermaterial (27) der ersten Öffnung (11) und/oder der zweiten Öffnung (13) gegenüberliegend angeordnet ist; und/oder
wobei das Filtermaterial (27) als Teil eines in die Kammer (9) und/oder den Kanal (15) einschiebbaren Einschubelements (29) ausgebildet ist.

9. Atemtherapiegerät (1) nach Anspruch 7 oder 8 rückbezogen auf Anspruch 5,
wobei das Filtermaterial (27) am Kammerdeckel (31) befestigt ist und zusammen mit dem Kammerdeckel (31) abnehmbar und/oder beweglich gelagert ist.

10. Atemtherapiegerät (1) nach einem der vorhergehenden Ansprüche,
wobei die Kammer (9) ferner einen rohrförmigen weiteren Kanal (33) umfasst, wobei der weitere Kanal (33) einen von der zweiten Öffnung (13) ins Innere der Kammer (9) ragenden weiteren inneren Kanalabschnitt (33a) und/oder einen von der zweiten Öffnung (13) in die Außenumgebung der Kammer (9) ragenden weiteren äußeren Kanalabschnitt (33b) umfasst;
wobei das Atemtherapiegerät (1) so ausgebildet ist, dass das Atemgas, wenn es vom Gaseinlass (3) zum Gasauslass (5) gefördert wird, ferner den weiteren Kanal (33) passiert.

11. Atemtherapiegerät (1) nach Anspruch 10,
wobei ein offenes Ende des weiteren inneren Kanalabschnitts (33a) der ersten Öffnung (11) und/oder einem offenen Ende des inneren Kanalabschnitts (15a) in Richtung einer Strömung (21) des Atemgases durch die Kammer (9) betrachtet zumindest teilweise gegenüberliegt; und/oder
wobei ein offenes Ende des weiteren inneren Kanalabschnitts (33a) einen anderen Strömungsquerschnitt, insbesondere einen größeren Strömungsquerschnitt, als die erste Öffnung (11) und/oder als ein offenes Ende des inneren Kanalabschnitts (15a) hat; und/oder
wobei eine Länge des weiteren inneren Kanalabschnitts (33a) höchstens ein Drittel einer Länge des inneren Kanalabschnitts (15a) beträgt; und/oder
wobei der weitere innere Kanalabschnitt (33a) und der weitere äußere Kanalabschnitt (33b) eine gemeinsame Längsachse (L) haben; und/oder
wobei ein Strömungsquerschnitt des weiteren inneren Kanalabschnitts (33a) in seiner Größe und/oder Form mit einem Strömungsquerschnitt des weiteren äußeren Kanalabschnitts (33b) übereinstimmt.

12. Atemtherapiegerät (1) nach Anspruch 10 oder 11,
wobei der Kanal (15) einen anderen Strömungsquerschnitt, insbesondere einen kleineren Strömungsquerschnitt, als der weitere Kanal (33) hat; und/oder
wobei eine Länge des weiteren Kanals (33) höchstens ein Drittel einer Länge des Kanals (15) beträgt; und/oder
wobei der Kanal (15) und der weitere Kanal (33) eine gemeinsame Längsachse (L) haben.

13. Atemtherapiegerät (1) nach einem der vorhergehenden Ansprüche, ferner umfassend:
eine zusätzliche Kammer (35) zur Schalldämpfung, wobei die zusätzliche Kammer (35) eine zusätzliche erste Öffnung (37), eine zusätzliche zweite Öffnung (39) und einen rohrförmigen zusätzlichen Kanal (41) umfasst, wobei der zusätzliche Kanal (41) einen von der zusätzlichen ersten Öffnung (37) ins Innere der zusätzlichen Kammer (35) ragenden zusätzlichen inneren Kanalabschnitt (41a) und/oder einen von der zusätzlichen ersten Öffnung (37) in eine Außenumgebung der zusätzlichen Kammer (35) ragenden zusätzlichen äußeren Kanalabschnitt (41b) umfasst;
wobei das Atemtherapiegerät (1) so ausgebildet ist, dass das Atemgas, wenn es vom Gaseinlass (3) zum Gasauslass (5) gefördert wird, ferner zwischen der zusätzlichen ersten Öffnung (37) und der zusätzlichen zweiten Öffnung (39) durch die zusätzliche Kammer (35) strömt und dabei den zusätzlichen Kanal (41) passiert.

## Claims

1. A respiratory therapy apparatus (1), comprising:
a gas inlet (3);
a gas outlet (5);
a blower (7) for conveying respiratory gas from the gas inlet (3) to the gas outlet (5);
a chamber (9) for sound attenuation, wherein the chamber (9) comprises a first opening (11), a second opening (13), and a tubular channel (15), wherein the channel (15) comprises an inner channel section (15a) projecting from the first opening (11) into the interior of the chamber (9) and an outer channel section (15b) projecting from the first opening (11) into an external environment of the chamber (9), wherein the second opening (13), as viewed in the direction of a flow (21) of the respiratory gas through the chamber (9), is at least partially opposite an open end of the inner channel section (15a), wherein the chamber (9) is bounded in a longitudinal direction (x) on one side by a first wall (17) and on the other side by a second wall (19), wherein the first wall (17) has the first opening (11) and the second wall (19) has the second opening (13), wherein a first end of the inner channel section (15a) is connected to the first wall (17) and a second, unconnected end of the inner channel section (15a) projects into the interior of the chamber (9) as the open end of the inner channel section (15a);
wherein the respiratory therapy apparatus (1) is designed such that, when the respiratory gas is conveyed from the gas inlet (3) to the gas outlet (5), the respiratory gas flows through the chamber (9) from the second opening (13) to the first opening (11) and, in doing so, passes through the channel (15);
wherein the second opening (13) is a passage through the second wall (19), wherein the passage connects an inner surface of the second wall (19), said inner surface facing the interior of the chamber (9), to an outer surface of the second wall (19), said outer surface facing the external environment of the chamber (9), wherein the second opening (13) has a larger flow cross section than the open end of the inner channel section (15a);
**characterized in that** the second opening (13) terminates flush with both the inner surface of the second wall (19) and the outer surface of the second wall (19).

2. The respiratory therapy apparatus (1) according to claim 1,
wherein the second opening (13) furthermore, when viewed in the direction of the flow (21) of the respiratory gas through the chamber (9), is at least partially opposite the first opening (11); and/or
wherein the second opening (13) furthermore has a different flow cross section, in particular a larger flow cross section, than the first opening (11).

3. The respiratory therapy apparatus (1) according to one of the preceding claims,
wherein the size and/or shape of a flow cross section of the inner channel section (15a) corresponds to a flow cross section of the outer channel section (15b); and/or
wherein the inner channel section (15a) and the outer channel section (15b) have a common longitudinal axis (L).

4. The respiratory therapy apparatus (1) according to one of the preceding claims,
wherein the inner channel section (15a) projects into the interior of the chamber (9) by at most up to half of a length of the chamber (9) in the longitudinal direction (x) between the first wall (17) and the second wall (19).

5. The respiratory therapy apparatus (1) according to one of the preceding claims,
wherein the chamber (9) further comprises a removable and/or movably mounted chamber cover (31) for closing the chamber (9).

6. The respiratory therapy apparatus (1) according to claim 5,
wherein the chamber cover (31), when it closes the chamber (9), forms at least one section of the first wall (17) and/or of the second wall (19).

7. The respiratory therapy apparatus (1) according to one of the preceding claims,
wherein the chamber (9) further comprises a filter material (27) for filtering particles and/or moisture from the respiratory gas flowing through the chamber (9) between the first opening (11) and the second opening (13).

8. The respiratory therapy apparatus (1) according to claim 7,
wherein the filter material (27) at least partially fills the interior of the chamber (9) and/or at least partially fills the channel (15); and/or
wherein the filter material (27) is disposed opposite the first opening (11) and/or the second opening (13); and/or
wherein the filter material (27) is designed as part of an insertion element (29) insertable into the chamber (9) and/or the channel (15).

9. The respiratory therapy apparatus (1) according to claim 7 or 8 related to claim 5,
wherein the filter material (27) is fastened to the chamber cover (31) and, together with the chamber cover (31), is removable and/or movably mounted.

10. The respiratory therapy apparatus (1) according to one of the preceding claims,
wherein the chamber (9) further comprises a tubular further channel (33), wherein the further channel (33) comprises a further inner channel section (33a) projecting from the second opening (13) into the interior of the chamber (9) and/or a further outer channel section (33b) projecting from the second opening (13) into the outer environment of the chamber (9);
wherein the respiratory therapy apparatus (1) is designed such that, when the respiratory gas is conveyed from the gas inlet (3) to the gas outlet (5), the respiratory gas further passes through the further channel (33).

11. The respiratory therapy apparatus (1) according to claim 10,
wherein, viewed in a direction of a flow (21) of the respiratory gas through the chamber (9), an open end of the further inner channel section (33a) is at least partially opposite the first opening (11) and/or an open end of the inner channel section (15a); and/or
wherein an open end of the further inner channel section (33a) has a different flow cross section, in particular a larger flow cross section, than the first opening (11) and/or than an open end of the inner channel section (15a); and/or
wherein a length of the further inner channel section (33a) is at most one third of a length of the inner channel section (15a); and/or
wherein the further inner channel section (33a) and the further outer channel section (33b) have a common longitudinal axis (L); and/or
wherein the size and/or shape of the flow cross section of the further inner channel section (33a) corresponds to the flow cross section of the further outer channel section (33b).

12. The respiratory therapy apparatus (1) according to claim 10 or 11,
wherein the channel (15) has a different flow cross section, in particular a smaller flow cross section, than the further channel (33); and/or
wherein the length of the further channel (33) is at most one third of the length of the channel (15); and/or
wherein the channel (15) and the further channel (33) have a common longitudinal axis (L).

13. The respiratory therapy apparatus (1) according to one of the preceding claims, further comprising:
an additional chamber (35) for sound attenuation, wherein the additional chamber (35) comprises an additional first opening (37), an additional second opening (39), and a tubular additional channel (41), wherein the additional channel (41) comprises an additional inner channel section (41a) projecting from the additional first opening (37) into the interior of the additional chamber (35) and/or an additional outer channel section (41b) projecting from the additional first opening (37) into an exterior environment of the additional chamber (35);
wherein the respiratory therapy apparatus (1) is designed such that, when the respiratory gas is conveyed from the gas inlet (3) to the gas outlet (5), the respiratory gas further flows through the additional chamber (35) between the additional first opening (37) and the additional second opening (39) and, in doing so, passes through the additional channel (41).

## Revendications

1. Appareil de thérapie respiratoire (1), comprenant :
une entrée de gaz (3) ;
une sortie de gaz (5) ;
un ventilateur (7) destiné à acheminer du gaz respiratoire depuis l'entrée de gaz (3) vers la sortie de gaz (5) ;
une chambre (9) destinée à l'insonorisation, la chambre (9) comprenant une première ouverture (11), une deuxième ouverture (13) et un conduit en forme de tube (15), le conduit (15) comprenant une section de conduit interne (15a) faisant saillie depuis la première ouverture (11) vers l'intérieur de la chambre (9) et une section de conduit externe (15b) faisant saillie depuis la première ouverture (11) vers un environnement extérieur de la chambre (9), la deuxième ouverture (13) étant, vue dans la direction d'un écoulement (21) du gaz respiratoire à travers la chambre (9), disposée au moins partiellement en face d'une extrémité ouverte de la section de conduit interne (15a), la chambre (9) étant délimitée, dans une direction longitudinale (x), d'une part par une première paroi (17) et d'autre part par une deuxième paroi (19), la première paroi (17) comprenant la première ouverture (11) et la deuxième paroi (19) comprenant la deuxième ouverture (13), une première extrémité de la section de conduit interne (15a) étant reliée à la première paroi (17) et une deuxième extrémité non reliée de la section de conduit interne (15a) faisant saillie à l'intérieur de la chambre (9), en tant qu'extrémité ouverte de la section de conduit interne (15a) ;
dans lequel l'appareil de thérapie respiratoire (1) est conçu de telle sorte que le gaz respiratoire, lorsqu'il est acheminé de l'entrée de gaz (3) vers la sortie de gaz (5), s'écoule de la deuxième ouverture (13) vers la première ouverture (11) à travers la chambre (9), tout en passant par le conduit (15) ;
dans lequel la deuxième ouverture (13) constitue un passage à travers la deuxième paroi (19), le passage reliant une surface intérieure de la deuxième paroi (19), tournée vers l'intérieur de la chambre (9), à une surface extérieure de la deuxième paroi (19), tournée vers l'environnement extérieur de la chambre (9), la deuxième ouverture (13) présentant une section d'écoulement plus grande par rapport à l'extrémité ouverte de la section de conduit interne (15a) ;
**caractérisé en ce que** la deuxième ouverture (13) affleure à la fois la surface intérieure de la deuxième paroi (19) et la surface extérieure de la deuxième paroi (19).

2. Appareil de thérapie respiratoire (1) selon la revendication 1,
dans lequel la deuxième ouverture (13), vue dans la direction d'écoulement (21) du gaz respiratoire à travers la chambre (9), est en outre au moins partiellement disposée en face de la première ouverture (11) ; et/ou
la deuxième ouverture (13) présente en outre une section d'écoulement différente, en particulier une section d'écoulement plus grande, par rapport à la première ouverture (11).

3. Appareil de thérapie respiratoire (1) selon l'une des revendications précédentes,
dans lequel une section d'écoulement de la section de conduit interne (15a) correspond, par sa taille et/ou sa forme, à une section d'écoulement de la section de conduit externe (15b) ; et/ou
dans lequel la section de conduit interne (15a) et la section de conduit externe (15b) présentent un axe longitudinal commun (L).

4. Appareil de thérapie respiratoire (1) selon l'une des revendications précédentes,
dans lequel la section de conduit interne (15a) fait saillie à l'intérieur de la chambre (9) sur une distance n'excédant pas la moitié d'une longueur de la chambre (9) dans la direction longitudinale (x) entre la première paroi (17) et la deuxième paroi (19).

5. Appareil de thérapie respiratoire (1) selon l'une des revendications précédentes,
dans lequel la chambre (9) comprend en outre un couvercle de chambre (31) amovible et/ou monté de façon mobile, destiné à fermer la chambre (9).

6. Appareil de thérapie respiratoire (1) selon la revendication 5,
dans lequel le couvercle de chambre (31), lorsqu'il ferme la chambre (9), constitue au moins une section de la première paroi (17) et/ou de la deuxième paroi (19).

7. Appareil de thérapie respiratoire (1) selon l'une des revendications précédentes,
dans lequel la chambre (9) comprend en outre un matériau filtrant (27) destiné à filtrer des particules et/ou de l'humidité à partir du gaz respiratoire s'écoulant à travers la chambre (9) entre la première ouverture (11) et la deuxième ouverture (13).

8. Appareil de thérapie respiratoire (1) selon la revendication 7,
dans lequel le matériau filtrant (27) remplit au moins partiellement l'intérieur de la chambre (9) et/ou remplit au moins partiellement le conduit (15) ; et/ou
dans lequel le matériau filtrant (27) est disposé en face de la première ouverture (11) et/ou de la deuxième ouverture (13) ; et/ou
dans lequel le matériau filtrant (27) est réalisé en tant que partie d'un élément d'insertion (29) pouvant être inséré dans la chambre (9) et/ou dans le conduit (15).

9. Appareil de thérapie respiratoire (1) selon la revendication 7 ou 8 dépendant de la revendication 5,
dans lequel le matériau filtrant (27) est fixé sur le couvercle de chambre (31) tout en étant amovible et/ou monté de façon mobile conjointement avec le couvercle de chambre (31).

10. Appareil de thérapie respiratoire (1) selon l'une des revendications précédentes,
dans lequel la chambre (9) comprend en outre un conduit supplémentaire (33) en forme de tube, ledit conduit supplémentaire (33) comprenant une section de conduit interne supplémentaire (33a) faisant saillie à partir de la deuxième ouverture (13) vers l'intérieur de la chambre (9) et/ou une section de conduit externe supplémentaire (33b) faisant saillie à partir de la deuxième ouverture (13) vers l'environnement extérieur de la chambre (9) ;
dans lequel l'appareil de thérapie respiratoire (1) est conçu de telle sorte que le gaz respiratoire, lorsqu'il est acheminé de l'entrée de gaz (3) vers la sortie de gaz (5), passe en outre par le conduit supplémentaire (33).

11. Appareil de thérapie respiratoire (1) selon la revendication 10,
dans lequel une extrémité ouverte de la section de conduit interne supplémentaire (33a), vue dans la direction d'un écoulement (21) du gaz respiratoire à travers la chambre (9), est disposée au moins partiellement en face de la première ouverture (11) et/ou d'une extrémité ouverte de la section de conduit interne (15a) ; et/ou
dans lequel une extrémité ouverte de la section de conduit interne supplémentaire (33a) présente une section d'écoulement différente, en particulier une section d'écoulement plus grande, par rapport à la première ouverture (11) et/ou à une extrémité ouverte de la section de conduit interne (15a) ; et/ou
dans lequel la longueur de la section de conduit interne supplémentaire (33a) n'excède pas un tiers de la longueur de la section de conduit interne (15a) ; et/ou
dans lequel la section de conduit interne supplémentaire (33a) et la section de conduit externe supplémentaire (33b) présentent un axe longitudinal commun (L) ; et/ou
dans lequel une section d'écoulement de la section de conduit interne supplémentaire (33a) correspond, par sa taille et/ou sa forme, à une section d'écoulement de la section de conduit externe supplémentaire (33b).

12. Appareil de thérapie respiratoire (1) selon la revendication 10 ou 11,
dans lequel le conduit (15) présente une section d'écoulement différente, en particulier une section d'écoulement plus petite, par rapport au conduit supplémentaire (33) ; et/ou
dans lequel la longueur du conduit supplémentaire (33) n'excède pas un tiers de la longueur du conduit (15) ; et/ou
dans lequel le conduit (15) et le conduit supplémentaire (33) présentent un axe longitudinal commun (L).

13. Appareil de thérapie respiratoire (1) selon l'une au moins des revendications précédentes, comprenant en outre :
une chambre supplémentaire (35) destinée à l'insonorisation, la chambre supplémentaire (35) comprenant une première ouverture supplémentaire (37), une deuxième ouverture supplémentaire (39) et un conduit supplémentaire en forme de tube (41), le conduit supplémentaire (41) comprenant une section de conduit interne supplémentaire (41a) faisant saillie à partir de la première ouverture supplémentaire (37) vers l'intérieur de la chambre supplémentaire (35) et/ou une section de conduit externe supplémentaire (41b) faisant saillie à partir de la première ouverture supplémentaire (37) vers un environnement extérieur de la chambre supplémentaire (35) ;
dans lequel l'appareil de thérapie respiratoire (1) est conçu de telle sorte que le gaz respiratoire, lorsqu'il est acheminé de l'entrée de gaz (3) vers la sortie de gaz (5), s'écoule en outre entre la première ouverture supplémentaire (37) et la deuxième ouverture supplémentaire (39) à travers la chambre supplémentaire (35), tout en passant par le conduit supplémentaire (41).
